# EUROPEAN PATENT SPECIFICATION

(11) **EP 3 229 727 B1**
(45) Date of publication and mention of the grant of the patent: **23.05.2018**
(21) Application number: 15808465.7
(22) Date of filing: 02.12.2015
(51) Int. Cl.: A61F 2/01

(54) **ENDOLUMINAL FILTER DESIGN VARIATIONS**
VARIATIONEN VON ENTWÜRFEN FÜR ENDOLUMINALE FILTER
VARIATIONS DE CONCEPTION DE FILTRE ENDOLUMINAL

(30) Priority: 11.12.2014 US 201462090580 P
(43) Date of publication of application: 18.10.2017
(73) Proprietor: Koninklijke Philips N.V., 5656 AE Eindhoven (NL); Volcano Corporation, San Diego, CA 92130 (US)
(72) Inventor: JOHNSON, Eric, 5656 AE Eindhoven (NL); STIGALL, Jeremy, 5656 AE Eindhoven (NL); PHILIPS, Jason, 5656 AE Eindhoven (NL)
(74) Representative: Steffen, Thomas
(86) International application number: PCT/IB2015/059273
(87) International publication number: WO 2016/092423

(56) References cited:
- WO-A2-02/089869
- WO-A2-2006/074163
- US-A1- 2003 212 429
- US-A1- 2004 153 117
- US-A1- 2010 217 381
- US-A1- 2015 164 630

## Description

### CROSS REFERENCE TO RELATED APPLICATIONS

This application is related to U.S. Patent Application No. 11/969,827, filed on January 4, 2008, titled "ENDOLUMINAL FILTER WITH FIXATION," and published as U.S. Patent Application Publication No. 2008/0147111 and U.S. Provisional Patent Application No. 61/917,865, filed on December 18, 2013, and tilted "FILTER SUPPORT MEMBERS,".

### FIELD

This invention relates generally to devices for providing filtration of debris within a body lumen. More particularly, the invention provides a retrievable filter placed percutaneously in the vasculature of a patient to prevent passage of emboli.

### BACKGROUND

Embolic protection is utilized throughout the vasculature to prevent the potentially fatal passage of embolic material in the bloodstream to smaller vessels where it can obstruct blood flow. The dislodgement of embolic material is often associated with procedures which open blood vessels to restore natural blood flow such as stenting, angioplasty, arthrectomy, endarterectomy or thrombectomy. Used as an adjunct to these procedures, embolic protection devices trap debris and provide a means for removal for the body.

One widely used embolic protection application is the placement of filtration means in the vena cava. Vena cava filters (VCF) prevent the passage of thrombus from the deep veins of the legs into the blood stream and ultimately to the lungs. This condition is known as deep vein thrombosis (DVT), which can cause a potentially fatal condition known as pulmonary embolism (PE).

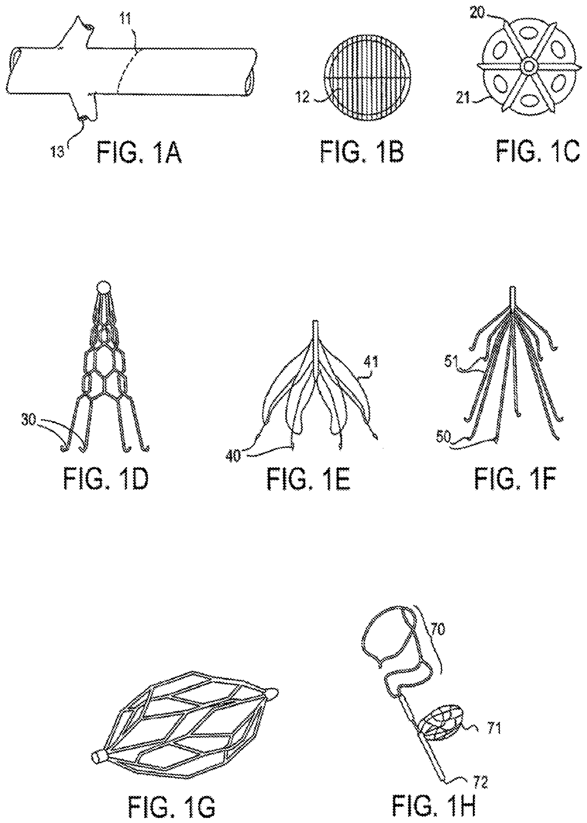

The first surgical treatment for PE, performed by John Hunter in 1874, was femoral vein ligation. The next major advancement, introduced in the 1950's, was the practice of compartmentalizing of the vena cava using clips, suture or staples. While effective at preventing PE, these methods were associated with significant mortality and morbidity (see, e.g., Kinney TB, Update on inferior vena cava filters, JVIR 2003; 14:425-440,). A major improvement in PE treatment, in which venous blood flow was maintained, was presented by DeWesse in 1955. This method was called the "harp-string" filter, as represented in FIG. 1A and FIG. 1B, in which strands of silk suture 12 were sewn across the vena cava 11 in a tangential plane below the renal veins 13 to trap thrombus. Reported clinical results demonstrated the effectiveness of this method in preventing PE and maintaining caval patency. (see, e.g., DeWeese MS, A vena cava filter for the prevention of pulmonary embolism, Arch of Surg 1963; 86:852-868,). Operative mortality associated with all of these surgical treatments remained high and therefore limited their applicability.

The current generation of inferior vena cava (IVC) filters began in 1967 with the introduction of the Mobin-Uddin umbrella 21 (FIG. 1C) which is described in further detail in U.S. Pat. No. 3,540,431. The Greenfield filter (FIG. 1D) was introduced in 1973 and is described in further detail in U.S. Pat. No. 3,952,747. These conical-shaped devices were placed endoluminaly in the IVC and utilized hooks or barbs 20, 30 to pierce the IVC wall and fix the position of the device. A variety of conical-shaped, percutaneously placed vena cava filters, based upon this concept are now available. For example, the TULIP with a filter structure 41 (FIG. 1E) further described in U.S. Pat. No. 5,133,733; the RECOVERY with a filter structure 51 (FIG. 1F) further described in U.S. Pat. No. 6,258,026; and the TRAPESE with a filter structure 61 (FIG. 1G) further described in U.S. Pat. No.6,443,972.

The next advancement in filters added the element of recoverability. Retrievable filters were designed to allow removal from the patient subsequent to initial placement. Retrievable filters are generally effective at preventing PE yet they have a number of shortcomings, such as, for example: failure of the device to deploy into the vessel properly, migration, perforation of the vessel wall, support structure fracture, retrievability actually limited to specific circumstances, and formation of thrombosis on or about the device.

Problems associated with retrievable, conical-shaped devices, such as those illustrated in FIG. 1D, FIG. 1E and FIG. 1F, have been reported in the medical literature. These reported problems include tilting, which makes it difficult to recapture the device and compromises filtration capacity. Hooks 30, 40, 50, 60 used to secure these devices have been reported to perforate the vessel wall, cause delivery complications, and fracture. A partially retrievable system is described in detail in pending U.S. Pat. No. 2004/0186512 (FIG. 1H). In this system, the filter portion 71 can be removed from the support structure 70, but the support structure remains in-vivo. All of these described devices share the common limitation that they can be retrieved from only one end.

US 2003/212429 A1 discloses in Fig. 30 a filter that has two axially spaced-apart support hoops which are interconnected by connecting arms.

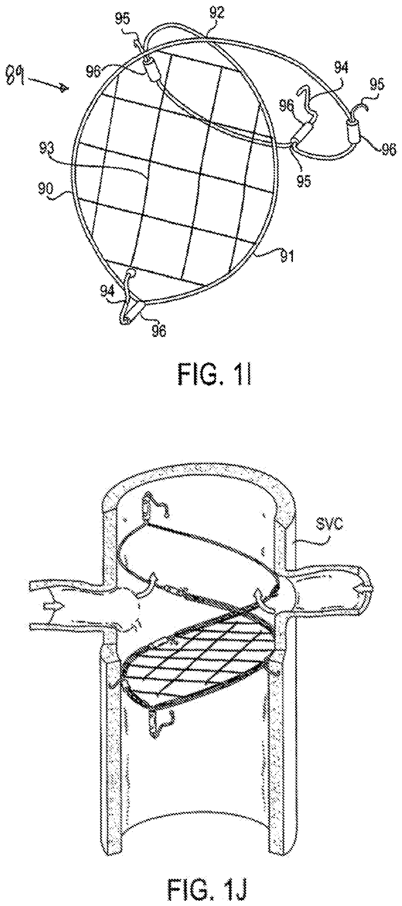

Additional retrievable endoluminal filters are disclosed in US 2008/0147111 to Eric Johnson et al. (FIG. 1I). FIG. 1I is a perspective view of an endoluminal filter 89 having a first support member 90 having a first end and a second end and a second support member 91 attached to the first end of the first support member 90 or the second end of the first support member 91. The second support member 91 forms a crossover 92 with the first support member 90. The second support member 91 and the first support member 90 are movable relative to each other at the crossover point. The first support member 90 and second support member 91 are movable relative to each other at crossover 92. A material capture structure 93 extends between the first and second support members 90, 91, the crossover 92, and the first end or the second end of the first support member 90. The filter illustrated in FIG. 1I has a retrieval feature 94 on the first end and a retrieval feature 94 on the second end. FIG. 1I illustrates a tissue anchor 95 on the first support member 90 and on the second support member 91. The tissue anchor 95 can improve engagement with a luminal wall. The first support member 90 and second support member 91 can be joined together with a crimp 96.

FIG. 1J illustrates an endoluminal filter deployed in the superior vena cava (SVC). In view of the many shortcomings and challenges that remain in the field of endoluminal filtering, there remains a need for improved retrievable, endoluminal filters.

### SUMMARY OF THE DISCLOSURE

The invention is defined by claim1. The endoluminal filters can include a first support member having a first end and a second end, a second support member attached to the first end of the first support member or the second end of the first support member and with the first support member fixed to the second support member, a material capture structure extending between the first and second support members and the first end or the second end of the first support member, and at least one tissue anchor on the first support member or the second support member.

In any of the embodiments disclosed herein the filter can have a first loop formed between the first support member and second support member and a second loop formed between the first support member and second support member.

In any of the embodiments disclosed herein the first support member does not crossover the second support member.

In any of the embodiments disclosed herein the second support member is attached to the first end of the first support member and the second end of the first support member.

In any of the embodiments disclosed herein the first support member and the second support member are formed from a single wire.

In any of the embodiments disclosed herein the first support member forms the tissue anchor and the second support member forms a retrieval feature.

In any of the embodiments disclosed herein the filter further includes a retrieval feature on the first end and a retrieval feature on the second end.

In any of the embodiments disclosed herein the at least one tissue anchor is formed on the surface of the first support member or the second support member.

In any of the embodiments disclosed herein the tissue anchor is formed from or attached to a tube covering at least a portion of the first support member or the second support member. According to the invention the first support member and second support member comprise a microtruss structure having a metallic lattice structure.

In any of the embodiments disclosed herein the first support member and second support member comprise one or more divots configured to engage with the material capture structure.

In any of the embodiments disclosed herein the first support member and second support member include one or more openings configured to engage with the material capture structure. According to the invention the filters include a support frame with a first wall portion forming a first loop and a second wall portion forming a second loop a material capture structure extending between the first wall portion of the first loop, and at least one tissue anchor on the support frame. The first wall portion and second wall portion can be integral with the other. The first wall portion and second wall portion configured are configured such that the first wall portion does not crossover the second wall portion.

In any of the embodiments disclosed herein, the endoluminal filter further comprises a retrieval feature.

In any of the embodiments disclosed herein the at least one tissue anchor is formed on a surface of the first wall portion or second wall portion.

In any of the embodiments disclosed herein the support frame is laser machined from a tubular structure.

In any of the embodiments disclosed herein the support frame is made using a 3D printer.

In any of the embodiments disclosed herein the support frame comprises a substantially smooth outer surface.

In any of the embodiments disclosed herein the first wall portion and second wall portion include a roughened or jagged outer surface. According to the invention the support frame comprises a microtruss structure having a metallic lattice.

In any of the embodiments disclosed herein the first wall portion and second wall portion comprise one or more divots configured to engage with the material capture structure.

In any of the embodiments disclosed herein the first wall portion and second wall portion include one or more openings configured to engage with the material capture structure.

In any of the embodiments disclosed herein the first wall portion includes a sinusoidal shape. In any of the embodiments disclosed herein the second wall portion includes a sinusoidal shape. In any of the embodiments disclosed herein the material capture structure extends between the first wall portion having the sinusoidal shape.

In any of the embodiments disclosed herein the filter further includes a rigid connection member between the support frame and an opposing side of the support frame. In any of the embodiments disclosed herein the rigid connection member joins the support frame and opposing side of the support frame at a position where the distance between the support frame and opposing side of the support frame is at a minimum. In any of the embodiments disclosed herein further includes a retrieval member projecting from the rigid connection member.

In any of the embodiments disclosed herein further includes an elongate third portion between the first loop and second loop. In any of the embodiments disclosed herein the support frame further includes a third wall portion forming a third loop. In any of the embodiments disclosed herein the endoluminal filter is made out of a single material. Exemplary methods of positioning a filter within a lumen are provided. The methods can include advancing a sheath containing the filter of any of the preceding claims through the lumen, deploying a portion of the filter of any of the preceding claims from the sheath into the lumen to engage the lumen wall while maintaining substantially all of the material capture of the filter within the sheath, and deploying the material capture structure of the filter of any of the preceding claims from the sheath to a position across the lumen. The methods can further include maneuvering a snare towards the filter in the same direction used during the advancing step; and engaging the snare with the filter retrieval feature positioned against a wall of the lumen. The methods can further include maneuvering a snare towards the filter in the opposite direction used during the advancing step; and engaging the snare with the filter retrieval feature positioned against a wall of the lumen. The methods can further include deploying the filter retrieval feature from the sheath after the deploying the material capture structure step. The methods can further include deploying the filter retrieval feature from the sheath before the deploying the material capture structure step. Deploying a portion of the filter step further includes engaging the lumen wall with the tissue anchor. Deploying a portion of the filter step includes engaging the lumen wall with a radial force generated by the filter.

### BRIEF DESCRIPTION OF THE DRAWINGS

The novel features of the invention are defined in the claims that follow. A better understanding of the features and advantages of the present invention will be obtained by reference to the following detailed description that sets forth illustrative embodiments, in which the principles of the invention are utilized, and the accompanying drawings of which:
FIGs. 1A-1I illustrate various prior art filters. FIG. 1J illustrates a prior art filter deployed in the superior vena cava.
FIGs. 2A-2C illustrate various features of endoluminal filter devices.
FIGs. 3A-3C illustrate various features of endoluminal filter devices.
FIGs. 4A-4C illustrate various features of endoluminal filter devices.
FIGs. 5A-5C illustrate various features of endoluminal filter devices.
FIGs. 6A-6B illustrate various features of endoluminal filter devices.
FIG. 7 illustrates various features of an endoluminal filter device.
FIGs. 8A-8B illustrate various features of endoluminal filter devices.
FIG. 9 illustrates various features of an endoluminal filter device.
FIGs. 10A-10C illustrate various features of endoluminal filter devices.
FIGs. 11A-11C illustrate various features of endoluminal filter devices.
FIG. 12 illustrates various features of an endoluminal filter device.
FIGs. 13A-13B illustrate an embodiment of an endoluminal filter device made from a flat sheet.
FIGs. 14A-25F illustrate several alternative filtering structures.

### DETAILED DESCRIPTION

There remains a clinical need for improved endoluminal filter devices and methods. Improved endoluminal filter devices provide effective filtration over a range of lumen sizes and are easy to deploy into and retrieve from a lumen. In addition, improved endoluminal filter devices minimize thrombosis formation or tissue ingrowth on the device and are resistant to migration along the lumen. Embodiments of the filter devices disclosed herein provide many improved features. The endoluminal filters disclosed herein have a number of uses such but are not limited to: embolic protection, thrombectomy, vessel occlusion, and tethered or untethered distal protection.

Embodiments disclosed herein provide advantages over the prior art filters. For example, the filters disclosed herein can be manufactured with more complicated geometries using different production methods, such as additive manufacturing and laser cutting tubular and flat materials. Examples of additive manufacturing techniques including 3D printing, sintering, etc. More complicated geometries can be obtained with decreased assembly steps and with a decreased number of pieces used to assemble the filter. Decreasing the number of separate pieces used to manufacture the filter decreases the likelihood of the pieces separating or the filter breaking while implanted in the patient and causing serious complications.

3D printing processes also provide greater repeatability and reproducibility in comparison to prior art methods. 3D printing provides the ability to obtain geometries that are difficult or impossible with current manufacturing techniques. 3D printing can produce devices at a much lower manufacturing cost than current manufacturing techniques. The use of 3D printing can produce a filter with a lower profile, e.g. a filter without crimps or redundant material. 3D printing also makes it easy to produce filters with custom sizes and configurations. In one example, 3D printing can be used to make an entire filter out of a biodegradable polymer having nooks and crannies to be used for drug elution.

Some embodiments include manufacturing the filter out of a single integral material, as in 3D printing. For example, the filter support frame, material capture structure, retrieval feature, and optional anchor structure can all be made out of a single integral material.

Manufacturing the filter out of a single integral material using 3D printing avoids the additional processing steps required to assemble the wire structures used in the prior art. In addition, using a single integral material avoids the processing used to join and crimp the wire structures used in the prior art. Also, manufacturing using 3D printing is advantageous over processing metal wires as the desired shape can be made with 3D printing without having to clamp together or treat the wire ends.

Embodiments where the filter is made from a single integral material also offer advantages over prior art filters in that they are not assembled from multiple pieces that can become separated while in the patient thereby causing serious complications in the patient. The improved manufacturing efficiency decreases production time and costs while allowing for great flexibility in the manufactured filter structure. According to the invention support frames do not cross over another section of the support frame, in contrast to the prior art filter illustrated in FIG. 1I which has a crossover 92. The support frame walls can form a first loop and second loop similar to an infinity shape or the shape of the number 8 but without the crossover. The support frame has a shorter distance between the opposing frame members around a midpoint area between the first and second loops instead of a crossover. Filters configurations without a crossover eliminate wire on wire fretting abrasion. Filter configurations without a crossover also reduce the loading profile on the filter. Filter configurations without a crossover also can have a shorter length than filters with a crossover point. Filter configurations without a crossover are also simpler and easier to manufacture than filters with a crossover point.

In some examples disclosed herein the filter includes a crossover between the walls of the support frame with the walls fixed together at the crossover, in contrast to the prior art filter illustrated in FIG. 1I which has support members 90, 91 movable relative to each other at the crossover 92. The frame walls can be tied, crimped, welded, or held together by other mechanical structures. Filters having a fixed crossover can adjust to a broad range of lumen sizes and geometries. Filters having a fixed crossover can also decrease or eliminate sharp bends thereby increasing fatigue durability of the filter.

In some embodiments the adjacent frame walls that do not cross over can be fixed together around a midpoint adjacent to where the opposing filter walls are closest together. The opposing support frame walls can be fixed together at a fixation point around the midpoint using a mechanical structure.

The support frames disclosed herein can have different shapes and textures. In some embodiments the support frame can have a smooth texture. In some embodiments the support frame can have a roughened texture, such as small projections or a jagged exterior, which can contact the vessel wall to hold the filter in place.

In some embodiments the support frames can be modified to include filter attachment points. For example the support frame can have a hole, hook, loop, divot, or other structure to facilitate holding a filter element or part of a material capture structure. According to the invention the support frame is made out of a microtruss structure having a metallic lattice structure. The microtruss structure can offer improved strength over prior art filter materials. The microtruss structure can also provide openings that provide filter attachment points. The microtruss structure can be made from laser cutting, EDM, 3-D printing, and other suitable methods. The microtruss structures allow for greater customization of the support structure properties. For example, failure analysis can be used to identify areas that need to be more flexible or more rigid. The filter structure can then be modified to use a microtruss structure or different microtruss structure in the desired filter areas to increase the flexibility or rigidity of the filter. The microtruss structure can be used to carry a drug in the interstitial spaces of the structure. The microtruss structure can also have a lower profile with enhanced torsional flexibility, similar to a micro stent structure.

In some embodiments the support frames disclosed herein can be patterned or shaped to provide filter attachment points. For example the filter support frame can have a curved, coiled, or sinusoidal arrangement that can be used as filter attachment points. The ability to use portions of the support frame shape or pattern as filter attachment points can decrease the complexity of the filter design and greatly simplify the manufacturing process. This is a huge potential benefit.

Several embodiments provide improved filtration devices that are durable, provide effective and nearly constant filter capacity over a range of lumen sizes and are easily delivered and removed from a lumen. Additionally, embodiments can be delivered into and retrieved from a lumen using minimally invasive surgical techniques approaching either end of the filter.

In some embodiments the support frame is made using a shape memory material. The shape memory material may have a pre-shaped form that ensures the support elements are uniformly collapsible and, when deployed, provides a pre-defined range of controllable force against the lumen wall without use of hooks or barbs.

In some embodiments the filter frames disclosed herein can include at least one anchor or fixation point to promote atraumatic contact with the lumen walls. In some embodiments textured surfaces (e.g. roughed or jagged surface), hooks, barbs, or other fixation elements or devices may be used with any of the filter embodiments described herein. In some embodiments a portion of the truss structure or a zigzag structure can flare out to form a barb.

In some embodiments the material capture structure can include filter elements or parts of the frame walls. In some embodiments the material capture structure can extend tautly across the area between the frame walls. In some embodiments the material capture structure can have a basket configuration or windsock configuration.

The support frame can be configured to collapse and expand with natural vessel movements while maintaining constant apposition with the vessel wall. One result is that the support frame shape and size track to vessel movements. As a result, the filter density and capacity of embodiments of the present invention remain relatively independent of changes in vessel size. Moreover, the self-centering aspect of the support structure ensures the filtration device provides uniform filtration across the vessel diameter. As such, embodiments of the present invention provide generally constant filtration capacity of the device across the entire vessel lumen and during vessel contractions and expansions.

Uniform filter capacity is a significant improvement over some conventional devices. Conventional devices typically have a filter capacity that varies radially across a lumen. The radial variation in filter capacity usually results from the fact that conventional filtration elements have a generally wider spacing at the periphery of the lumen and closer spacing along the central lumen axis. The result is that larger emboli can escape along the lumen periphery.

During vessel expansions and contractions, the radial variations in filter capacity are exacerbated in conventional devices.

Another advantage of some embodiments is that when released from a constrained state (i.e., within a delivery sheath), the device assumes a pre-determined form with the support frame self-centering the device in the vessel. The support frame exerts atraumatic radial force against the vessel wall to prevent or minimize device migration. In some embodiments, radial forces generated by the support frame work in cooperation with an optionally textured support frame surface and hooks, barbs or other fixation devices to secure the device within the vessel. Hooks, barbs or other fixation devices or elements may be used as an added precaution against migration of the filtering device while in a lumen. When device retrieval is initiated, the uniformly collapsible form of the support frame causes the support frame to pull away from the vessel wall as the device is being re-sheathed. The movement of the support frame away from the vessel wall facilitates the atraumatic removal of the device from the vessel wall. Additionally, in those embodiments having hooks, barbs or other fixation devices or elements, elongate member movement during retrieval also facilitates withdrawal of the fixation elements from the lumen wall.

Additional embodiments of the present invention may include a retrieval feature on one or both ends of the device. The use of retrieval features on both ends of the device allows deployment, repositioning and removal of the device to be accomplished from either end of the device. As a result, the use of retrieval features on both ends of the device enables both antegrade or retrograde approaches to be used with a single device. The retrieval feature may be integral to another structural member or a separate component. In some embodiments, the retrieval feature is collapsible and may have a curved shape or a generally sinusoidal shape.

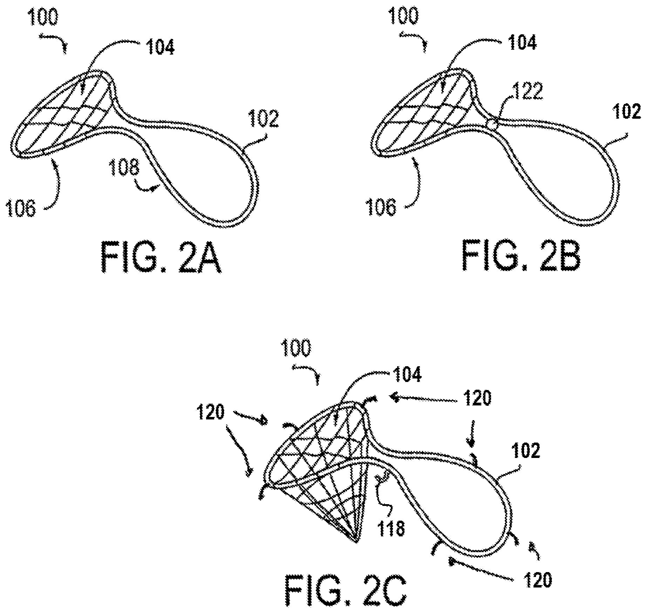

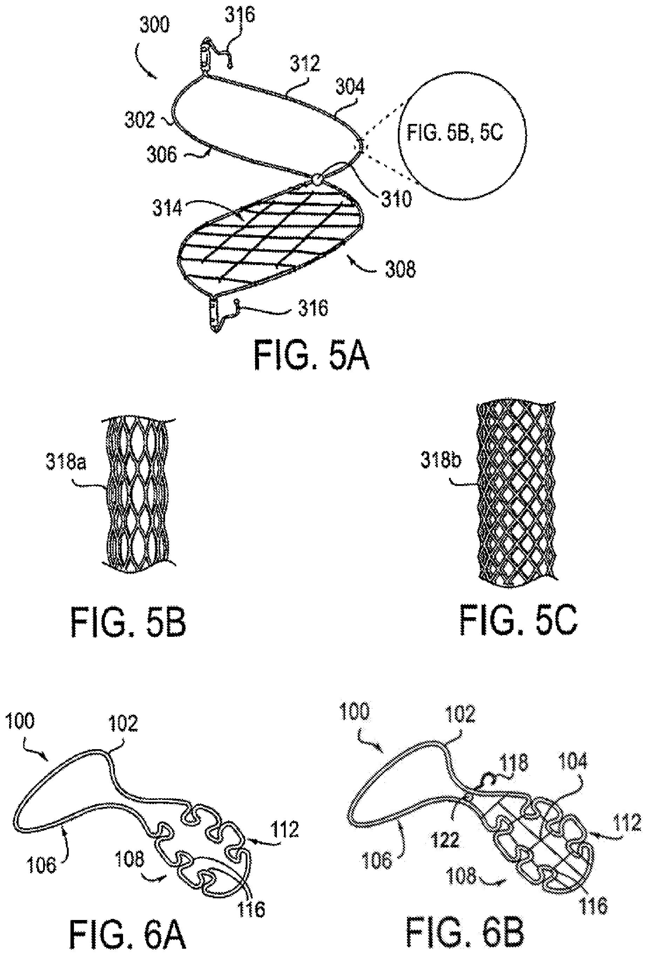

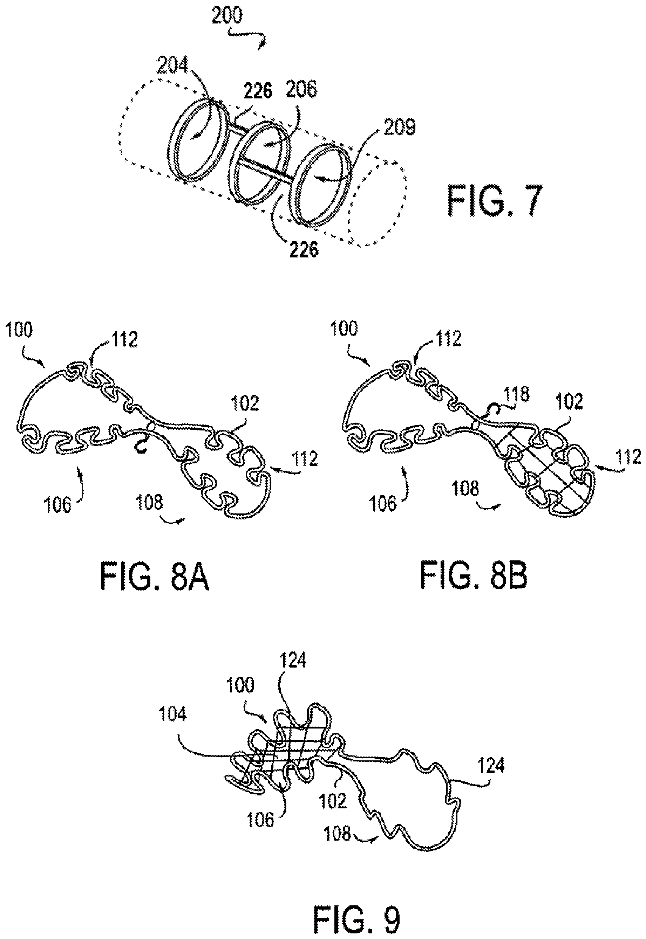

In some examples the filter has first and second support members with spiral structures that form a crossover point. The first and second support members are fixed together at the crossover point. The first and second support members can be welded together or fixed together using other types of mechanical structures. Examples of mechanical structures that can be used to fix the support members together at the crossover include: a rigid member, crimping, tying, rigid or elastic loop, clipping, etc. In some embodiments a rod or wire can pass through openings in the first and second support members adjacent to the crossover to fix the support members together. Examples of filters with fixed crossover points include FIGs. 5A, 11A-C, and 12. The fixed crossover point can help the filter keep the deployed shape and maintain improved apposition with the luminal walls. Filters having a fixed crossover can adjust to a broad range of lumen sizes and geometries. Filters having a fixed crossover can also decrease or eliminate sharp bends thereby increasing fatigue durability of the filter. According to the invention the support frames do not have or form a crossover point. In some embodiments the filter frames can resemble a barbell shape. In some embodiments the filter frames can resemble a figure 8 or infinity shape without a crossover. Other shapes are also possible. In some embodiments the filter frames form a first loop and a second loop. The loop can include a semi-circle, a substantially full circle, or a full circle. The loops can also include elliptical shapes. Examples of embodiments of filters that do not form crossover points include FIGs. 2A-2C, 3A-3B, 4A-4C, 6A-6B, 7, 8A-8B, and 9. Embodiments of filter frames without a crossover point can have additional flexibility over filters with crossover points.

Any of the support frames disclosed herein can include walls with sinusoidal bends. In some embodiments the sinusoidal bends can be in the plane defined by the loop, as illustrated in FIGs. 6A-6B and 8A-8B. In some embodiments the sinusoidal bends can be orthogonal to the plane defined by the loop, as illustrated in FIG. 9. The sinusoidal bends can be used to improve the overall strength of the filter, as filter attachment points, and to maintain improved contact with the lumen wall.

In some embodiments the filter frame walls can be fixed together. For example, the filter frame walls can be close together at a narrow region between the loop structures. The frame walls can be fixed together near the narrow region. Examples of mechanical structures that can be used to fix the support frame together include: a rigid member, crimping, tying, a rigid or elastic loop, welding, clipping, etc. Examples of embodiments of support frames with fixed walls that do not have a crossover are shown in FIGs. 5A, 2B, 6B, and 8A.

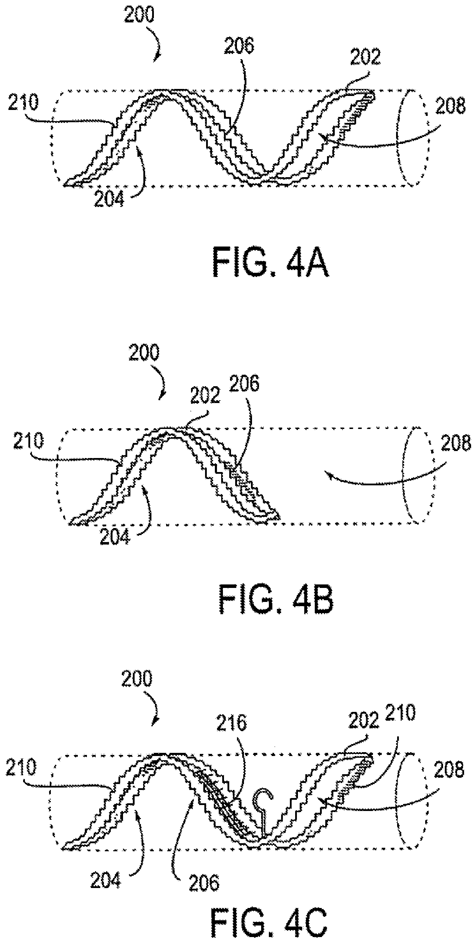

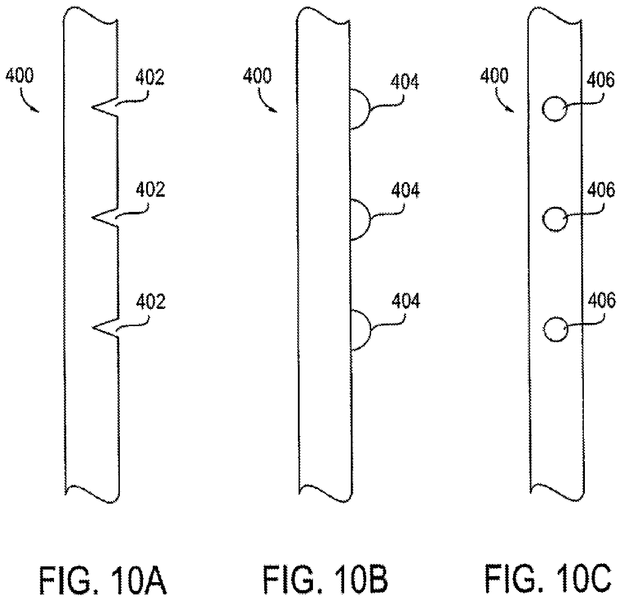

In some embodiments the support frame walls can be designed to include filter attachment points. For example the sinusoidal bends (FIG. 6B) can provide surfaces to attach the filter elements or material capture structure. The support frame walls can also be jagged or roughened as shown in FIG. 4A to provide a surface to secure or hold the filter elements or material capture structure to the support frame. Other examples of modifications to the support frame include divots as shown in FIG. 10A, loops as shown in FIG. 10B, and holes as shown in FIG. 10C.

In some embodiments any of the filter shapes disclosed herein can be made using 3D printing. In some embodiments portions of the filter, such as the support frame, are made using 3D printing with the material capture structure attached to the support frame. In some embodiments the entire filter structure is made using 3D printing include the support frame, material capture structure, optional retrieval features, and optional anchors. Examples of 3D printed filter structures are shown in FIGs. 2A-2C, 6A-6B, 8A-8B, and 9 and discussed in greater detail below.

In some embodiments 3D printing can be used to print two or more parts of any of the filters illustrated in FIGs. 1A-1J. For example, parts of the filters illustrated in FIGs. 1I and 1J can be 3D printed to improve manufacturing efficiency. In some examples the support members 90, 91 can be 3D printed such that they are movable relative to each other at the crossover 92. In some cases the relative motion between the support members 90, 91 at the crossover 92 can have movement limited in some way, such as by crimping or the other methods described herein. In some cases the material capture structure can be 3D printed with the support members or 3D printed and attached to the 3D printed support members. In some embodiments tissue anchors 95 and/or retrieval features 94 can be made by 3D printing. The use of 3D printing can eliminate the need for crimps 96 to attach these structures to the filter.

3D printing is advantageous because the final shape and structure is directly formed without the need to shape, bend, or attach anything to the structure. Shaping and processing the filter structure can causes stresses and weaken the structure. In addition attaching features to the structure takes additional processing time and increases the probability of the filter breaking while in the patient.

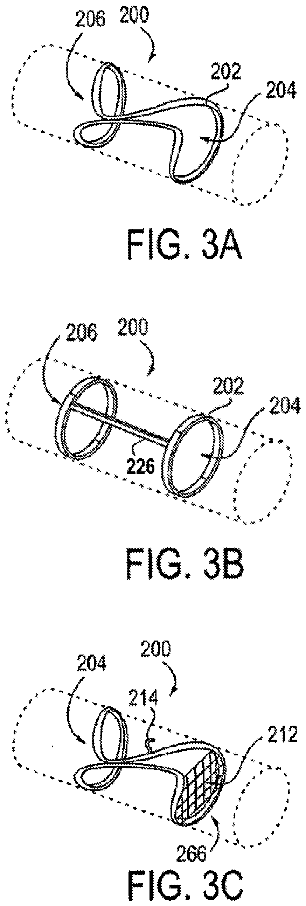

In some embodiments the filter structure is laser cut. The structure can be cut from a tubular structure or flat material. Examples of filter support frames laser cut from a tubular structure are shown in FIGs. 3A-3C, 4A-4C, and 7 and discussed in greater detail below. The dotted outlines in FIGs. 3A-3C, 4A-4C, and 7 refer to the tubular structure cut to form the filter structures. Laser cutting has the advantage of directly forming the filter support frame without the need to bend, shape, or assemble the frame. Additionally, wire ends don't have to be processed or held together.

In some embodiments the filter can carry a drug or medicine. For example, the filter can be configured such that the drug is eluted. The drug can be carried in nooks and crannies of the filter. The drug can also be embedded in interstitial areas of a microtruss structure. The filter can be provided with the drug present or can be configured to allow for a physician or medical technician to add the desired drug. The drug can be selected based on the desired treatment. In one example a drug is provided to promote the treatment of blood clots.

The filters disclosed herein can be made out of any biocompatible material. Examples of biocompatible materials include shape memory materials, biocompatible polymers, biodegradable polymers, and biocompatible materials suitable for 3D printing. In one embodiment, the support frame is formed from Magnetic Resonance Imaging (MRI) compatible materials. The support frame preferably contains no sharp bends or angles to produce stress risers that may lead to fatigue issues, vessel erosion, and facilitate device collapse.

Examples of suitable shape memory alloy materials include, for example, copper- zinc-aluminum, copper-aluminum-nickel, and nickel-titanium (NiTi or Nitinol) alloys. Shape memory polymers may also be used to form components of the filter device embodiments of the present invention. In general, one component, oligo(e-caprolactone) dimethacrylate, furnishes the crystallizable "switching" segment that determines both the temporary and permanent shape of the polymer. By varying the amount of the comonomer, n-butyl acrylate, in the polymer network, the cross-link density can be adjusted. In this way, the mechanical strength and transition temperature of the polymers can be tailored over a wide range. Additional details of shape memory polymers are described in US Patent 6,388,043. In addition, shape memory polymers could be designed to degrade. Biodegradable shape memory polymers are described in US Patent 6,160,084. Biodegradable polymers may also be used to form components of embodiments of the filter devices disclosed herein. For example, polylactide (PLA), a biodegradable polymer, has been used in a number of medical device applications including, for example, tissue screws, tacks, and suture anchors, as well as systems for meniscus and cartilage repair. A range of synthetic biodegradable polymers are available, including, for example, polylactide (PLA), polyglycolide (PGA), poly(lactide-co-glycolide) (PLGA), poly(e-caprolactone), polydioxanone, polyanhydride, trimethylene carbonate, poly(B-hydroxybutyrate), poly(g-ethyl glutamate), poly(DTH iminocarbonate), poly(bisphenol A iminocarbonate), poly(ortho ester), polycyanoacrylate, and polyphosphazene. Additionally, a number of biodegradable polymers derived from natural sources are available such as modified polysaccharides (cellulose, chitin, dextran) or modified proteins (fibrin, casein). The most widely compounds in commercial applications include PGA and PLA, followed by PLGA, poly(e-caprolactone), polydioxanone, trimethylene carbonate, and polyanhydride. Additional polymers that can be used include poly(amino acids) such as poly(L-glutamate), poly(L-lysine), and poly(L-leucine). Biodegradable polyurethane based materials can also be used.

In some embodiments non-polymeric materials can be used. In some embodiments non-shape memory materials are used. For example, magnesium and other biocompatible metals can also be used.

In some embodiments the filter structures disclosed herein can be made out of a single wire or multiple pieces of wire. According to the invention the support frame is made out of a material having a microtruss structure. An example of a microtruss structure is a metallic lattice structure. Examples of microtruss structures are shown in FIGs. 4B and 4C. Additional examples of microtruss structures include configurations used for stent structures. For example, the microtruss structure can resemble a diamond stent pattern, z-stent stent pattern, a sinusoidal pattern, a coiled stent pattern, a ribbon stent pattern, a braided pattern, etc. The microtruss structure could have a micro-sinusoidal pattern that forms a larger helical structure. Microtruss structures can be used in any of the filter embodiments disclosed herein. In some embodiments the microtruss structures can serve as filter attachment points. The microtruss structures offer increased strength while decreasing the density and weight of the overall device.

Any of the filter devices disclosed herein can be deployed and retrieved using minimally invasive catheter techniques. In some embodiments the material capture side of the filter is deployed first. In some embodiments the material capture side of the filter is deployed second. In some embodiments the deployed filter is captured using a retrieval feature on one of the ends of the filter. In some embodiments the filter is captured using a retrieval feature near a midpoint or fixation point on the filter. Contacting the retrieval feature on the filter can cause the filter to collapse. The collapsed filter can then be retrieved through the catheter.

The filter devices disclosed herein offer improved retrievability over prior art filter designs. The filter devices disclosed herein having a fixed crossover or lacking a crossover offer improvements over prior art filter devices with crossover features because the crossover feature can become endothelialized. Endothelialization can make filter removal more difficult. Additionally, the filter devices having a retrieval feature near a midpoint of the filter device can improve the collapsibility and symmetry for collapsing the filter.

Various features shown in the embodiments illustrated in the figures are now discussed. FIGs. 2A -2C illustrate various features of endoluminal filter devices in accordance with several embodiments. The filters 100 illustrated in FIGs. 2A-2C can be made from a single material using 3D printing.

FIG. 2A shows an endoluminal filter 100 with a support frame 102 forming a first loop 106 and second loop 108. A material capture structure 104 spans between the walls of the support frame that form the first loop 106. The filaments of the material capture structure 104 extend tautly between the support frame walls forming the first loop 106. The opposing support frame walls are closest together in the area between the first loop 104 and second loop 106. The support frame wall does not cross over the opposing wall.

FIG. 2B illustrates another embodiment of an endoluminal filter 100 with a support frame 102 forming a first loop 106 and second loop 108 with a material capture structure 104. The opposing support frame walls are joined by a rigid connector 122 adjacent to where the opposing support frame walls are closest together in the area between the first loop and second loop 106.

FIG. 2C illustrates another embodiment of an endoluminal filter 100. The endoluminal filter includes a support frame 102 with anchors 120 extending from the outer surface of the support frame. The anchors 120 are configured to engage with and hold the luminal wall tissue. The endoluminal filter 100 also includes a retrieval feature 118 extending from the support frame 102. The retrieval feature 118 is shown positioned between the first loop 106 and second loop 108; however, the retrieval feature can be positioned anywhere along the support frame. In contrast to FIGs. 2A and 2B, the endoluminal filter illustrated in FIG. 2C includes a material capture structure 104 that resembles a windsock.

FIGs. 3A-3C illustrate various embodiments of endoluminal filter devices that can be laser cut from a tube. FIG. 3A illustrates an endoluminal filter 200 with a support frame 202 forming a first loop 204 and a second loop 206. A material capture structure can extend between either or both of the first loop 204 and second loop 206. The dotted outline in FIG. 3A shows the outline of a tube that can be laser cut to form the endoluminal filter 200 shape and structure.

FIG. 3B illustrates an endoluminal filter 200 with a support frame 202 forming a first loop 204 and a second loop 206. FIG. 3B shows an elongated member 207 connecting the first loop 204 and second loop 206.

FIG. 3C illustrates an endoluminal filter 200 formed from a laser cut tube. The support frame 202 forms first loop 204 and second loop 206. A material capture structure 212 extends between the walls of the first loop 204. A retrieval feature 214 extends from the support frame 202 between the first loop 204 and the second loop 206.

FIGs. 4A-4C illustrate various embodiments of endoluminal filter devices that can be laser cut from a tube. The tube can be made out of a shape memory metal material. FIG. 4A illustrates a filter 200 with a support frame 202 forming a first loop 204, second loop 206, and third loop 209. Any or all of the first loop 204, second loop 206, and third loop 209 can include a material capture structure. The support frame 202 has an outer jagged structure 210 to facilitate apposition with the luminal walls. The jagged structure 210 can be used in place of anchors. The jagged structure 210 can also be used as filter attachment points.

The outer jagged structure is exaggerated in FIGs. 4A-4C. The support member can include smooth portions and a plurality of jagged structures to facilitate engagement with blood vessel walls and to optionally serve as material capture attachment points.

FIG. 4B illustrates a filter 200 with a support frame 202 forming a first loop 204 and second loop 206. Either or both of the first loop 204 and second loop 206 can include a material capture structure. The support frame 202 has an outer jagged structure 210.

FIG. 4C illustrates a filter 200 with a support frame 202 forming a first loop 204, second loop 206, and third loop 209. The second loop 206 includes a material capture structure 216. The material capture structure 216 can optionally span the first loop 204 and third loop 209 alone or in addition to the second loop 206. The support frame 202 has an outer jagged structure 210 to facilitate apposition with the luminal walls. A retrieval feature 214 is illustrated between the second loop 206 and third loop 209 although other positions on the frame are also possible.

FIGs. 4A-4C illustrate the support frame 202 with a jagged structure 210. Other alternatives to the illustrated jagged structure can be used. For example, the support frame can have a jagged structure in a plurality of location to facilitate engagement with the blood vessel walls and optionally as attachment points for the material capture structure. In other alternatives the support frame can be formed from the frame with a serpentine, sinusoidal, or other undulating shape. The sinusoidal or other undulating shape can facilitate the use of the filter in multiple blood vessel sizes.

FIGs. 5A-5C illustrate various features of endoluminal filter devices. FIG. 5A illustrates an endoluminal filter 300. The filter 300 includes a first support member 302 and second support member 304. The first support member 302 and second support member 304 form spiral structures that spiral in opposing directions. The first support member 302 forms a crossover point 310 with the second support member 304. In contrast to the filters disclosed in US2008/0147111, the first support member 302 is fixed to the second support member 306 at the crossover 310. The filter 300 includes a first loop 306 between the first support member 302, second support member 304, and crossover 310. The filter 300 includes a second loop 308 between the first support member 302, second support member 304, and crossover 310. A material capture structure 314 extends between the walls of the second loop 308. The support frame 312 can be made out of the microtruss structures illustrated in FIGs. 5B and 5C as 318a, 318b. The filter 300 includes retrieval features 316 on the filter end adjacent to the first loop 306 and the filter end adjacent to the second loop 308. FIGs. 5B-5C illustrate microtruss structures with metallic lattice patterns 318a, 318b.

Any of the filter designs disclosed herein can be made out of the microtruss structures illustrated in FIGs. 5B-5C. For example, any conventional wire structure or material used to make an endoluminal filter can be replaced by the microtruss structures illustrated in FIGs. 5B- 5C. In some examples the filter structures illustrated in FIGs. 1I and 1J can be produced with microtruss structures. In some cases the open metallic lattice patterns can serve as fixation points for the material capture structures.

FIGs. 6A-6B illustrate endoluminal filter devices having a coiled or sinusoidal wall configuration. The filters illustrated in FIGs. 6A-6B can be made using 3D printing. The filter 100 shown in FIG. 6A is similar to FIG. 2A but has a sinusoidal wall structure. The filter 100 includes a support frame 102 that forms a first loop 106 and a second loop 108. The second loop 108 has sinusoidal bends 112 instead of a smooth elliptical or circular shape. The sinusoidal bends 112 can include interior areas 116 that can serve as filter or material capture structure attachment points. In some embodiments the sinusoidal bends 112 can cover a greater portion of the second loop 108 area and function as the material capture structure.

FIG. 6B illustrates a filter 100 similar to FIG. 6A but with a retrieval feature 118 and a material capture structure 104. The material capture structure 104 extends between the interior areas 116 of the sinusoidal bends 112.

FIG. 7 illustrates various features of an endoluminal filter device laser cut from a tube. The filter 200 includes a first loop 204, second loop 206, and third loop 209. Rigid connectors 226 connect the first loop 204 to the second loop 206 and connect the second loop 206 to the third loop 209. The material capture structure can extend within any or all of the first loop 204, second loop 206, and third loop 209.

FIGs. 8A-8B illustrate additional endoluminal filter devices that can be manufactured using 3D printing. The filter 100 includes a support frame 102 that forms a first loop 106 and second loop 108. The first loop 106 and second loop 108 both have sinusoidal bends 112 instead of a smooth elliptical or circular shape. A retrieval feature 118 extends from the support frame. The frame walls are jointed together by the rigid connector 122.

FIG. 8B illustrates another embodiment of a filter 100 with sinusoidal bends 112 on the first loop 106 and second loop 108. A material capture structure extends between the sinusoidal bends of the first loop 106.

FIG. 9 illustrates various features of an endoluminal filter device 100. The filter 100 has a support frame 102 with a sinusoidal pattern 124. The bends illustrated in FIGs. 6A-6C and 8A-8B extend along the same plane defined by the first loop 106 and a plane formed by the second loop 108. In contrast to FIGs. 6A-6C and 8A-8B, the sinusoidal bends illustrated in FIG. 9 extend orthogonally to the plane formed by the first loop 106 and the plane formed by the second loop 108. The sinusoidal pattern 124 can serve as filter and material capture structure attachment points.

FIGs. 10A-10C illustrate various features of walls of endoluminal filter devices. FIG. 10A illustrates a cross-section of a support frame wall 400 having divots 402 configured to be filter or material capture structure attachment points. FIG. 10B illustrates a cross-section of a support frame wall 400 having loops 404 configured to be filter or material capture structure attachment points. FIG. 10C illustrates a cross-section of a support frame wall 400 having holes 406 configured to be filter or material capture structure attachment points.

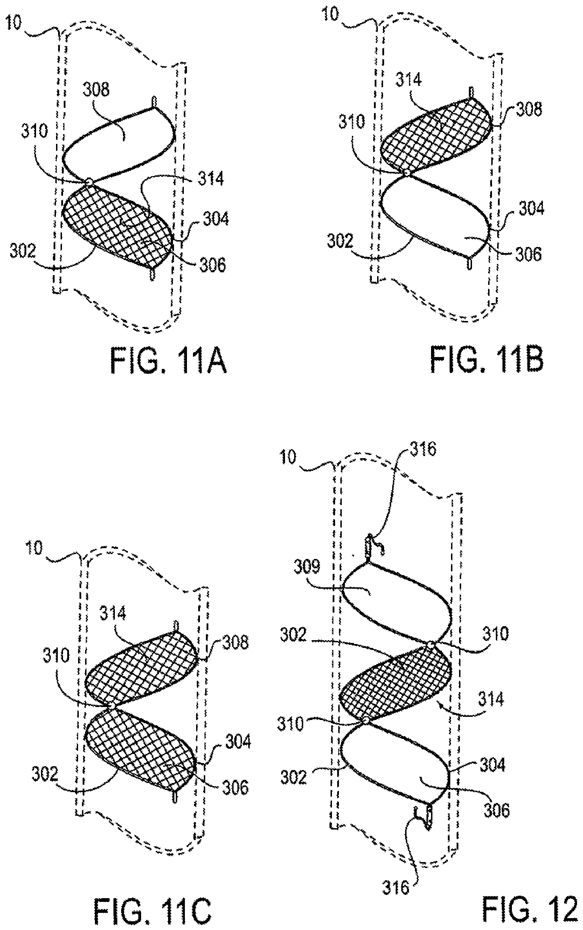

FIGs. 11A-11C illustrate endoluminal filter devices within a lumen 10. FIGs 11A-11C illustrate filters that include a first support member 302 and second support member 304 forming a first loop 306 and second loop 308. The first support member 302 has a ball 310 at the crossover point with the second support member

FIG. 11A has a material capture structure 314 extending within the first loop 306. FIG. 11B has a material capture structure 314 extending within the second loop 308. FIG. 11C has a material capture structure extending within the first loop 306 and the second loop 308. The ball 310 can constrain relative movement between the first support member 302 and the second support member 304.

FIG. 12 illustrates an endoluminal filter within a lumen 10. The filter include a first support member 302 and second support member 304 forming a first loop 306, second loop 308, and third loop 309. The first support member 302 forms has a ball 310 at each of the two crossover points between the second support member 304. A material capture structure 314 extends within the second loop 308. Retrieval features 316 are shown on the filter end adjacent to the first loop 306 and the filter end adjacent to the third loop 309. The ball 310 can constrain relative movement between the first support member 302 and the second support member 304.

In some examples the ball 310 can constrain the first and second support members such that they first and second support members are fixed together to restrict relative movement at the crossover point. The ball 310 can include two or more lumens to receive the first and second support members. The ball 310 can be crimped to the first and second support members.

In some cases the ball 310 is not completely fixed and instead allows some constrained motion between the first support member and second support member. For example the ball 310 could be lightly crimped to each of the first support member and second support member. In some cases the ball 310 can be crimped to one of the support members and allow movement of the other support member within the lumen of the ball 310. In some cases the lumen uses a rough material within the lumen to limit or restrict movements of the support members. Other structures are also possible in addition to or instead of the ball 310. For example a flexible joint, band, or dog bone shape can be used with one end on one support member and the other end on the other support member.

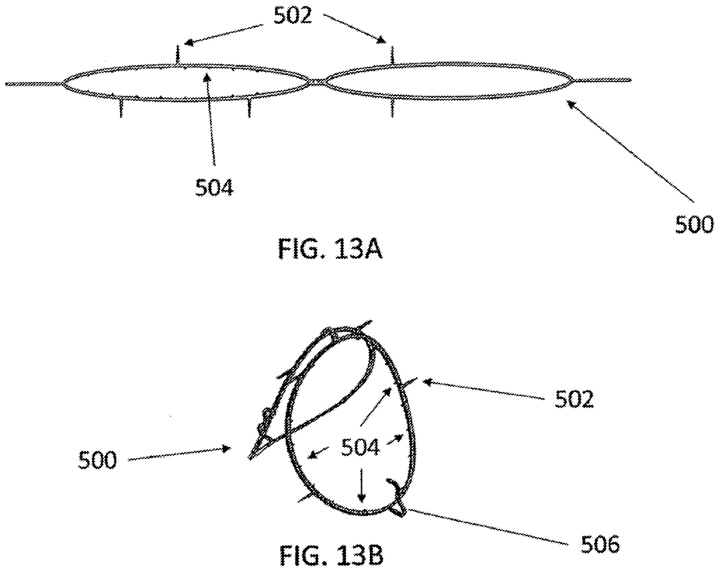

Any of the embodiments of endoluminal filters disclosed herein can be made from a flat material. For example, the filter can be made by laser cutting or laser machining the filter from a flat material. FIGs. 13A-13B illustrate an embodiment of an endoluminal filter made from a flat material. FIG. 13A illustrates the pre-expanded flat section of the laser cut pattern with the filter 500 having web eyelets 504 and side tissue anchors 502. FIG. 13B illustrates the post-expanded final version of the vena cava filter 500. The filter 500 includes anchors 502 and a retrieval feature 506. The radiopaque marker pockets and nitinol web are not illustrated. In some embodiments the material capture structure, such as a nitinol web, can be laser cut from the flat material. In some embodiments eyelets can be cut from the flat material to which the material capture structure can be secured.

Direct laser cutting of vena cava filters can accurately and reliably make a filter with a high resolution. Direct laser cutting expandable vena cava filters can produce filters with patterns having smooth, narrow cuts and very fine geometries. Laser cutting allows for the production of filters with integrated side tissue anchors, web eyelets and radiopaque marker pockets within a one-piece design. The laser cut pattern can also include a nitinol web integrated within the design in some embodiments. Direct laser cutting of the metal vena cava filter can enable greater precision, reliability, structural integrity, and overall quality of the filter, without burrs, slag or other imperfections which might otherwise hamper integrity and performance of the filter. The laser process conditions can be optimized based on the thickness of the material from which the filter is cut, the geometry of the material from which the filter is cut (flat sheet, tube, etc.), and geometry of the filter pattern. In some embodiments the laser can be precisely controlled, such as the laser power level, laser focus spot size, and the positioning of the laser cutting path. The filters can be cut from a filter with a fine precision structure can from a small or large diameter thin-walled cylindrical tube or a thin-walled flat sheet. Tubes and flat sheets can be made out of a metal or biocompatible polymer material. In some embodiments the tube or flat sheet can be made out a metal such as shape memory material like nitinol. The cylinder or flat material can be positioned under the laser utilizing a computer numerical control (CNC) system to cut a filter with an intricate and precise pattern.

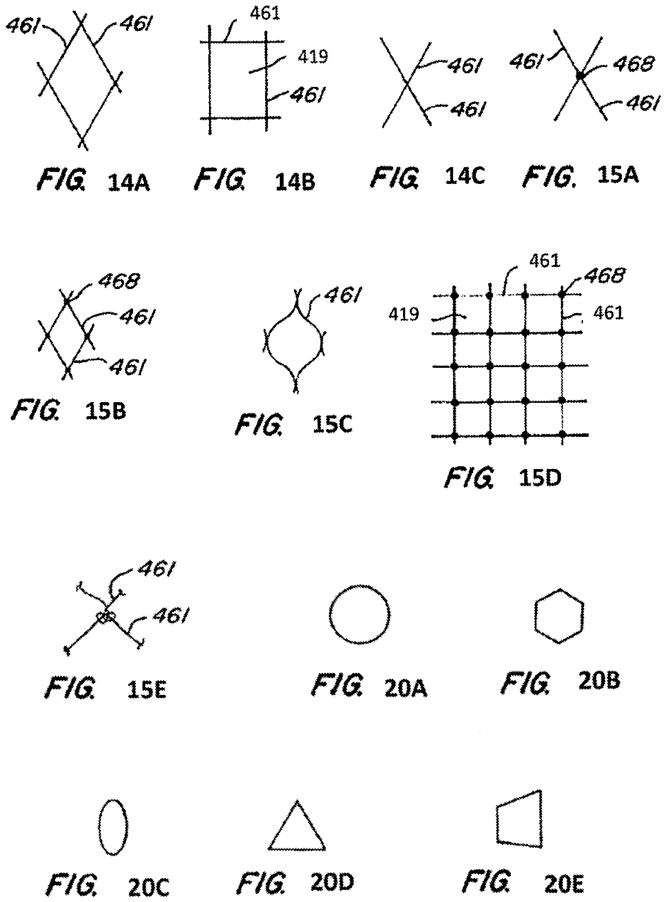

The material capture structures illustrated in Figures 14A-25F can be used in any of the embodiments of endoluminal filters described herein. The material capture structure can be selected based on the desired application for the filter. For example if the filter is for arterial and/or distal protection then a fine material capture structure could be used. In another example, if the filter is for embolic protection then the material capture structure can be occlusive.

In some embodiments, the material capture structure contains a number of filter cells. Filter cells may be formed in a number of different ways and have a number of different shapes and sizes. The shape, size and number of filter cells in a specific filter may be selected based on the use of a particular filter. For example, a filter device of the present invention configured for distal protection may have a filter cell size on the order of tens to hundreds of microns to less than 5 millimeters formed by a selecting a filter material with a pore size (FIGs. 15A, 15B) suited to the desired filtration level. In other applications, the filter cell may be formed by overlapping (i.e., joined or crossed without joining) filaments to form cells that will filter out debris in a lumen above a size of 2 mm. Various other filter sizes and filtration capacities are possible as described herein.

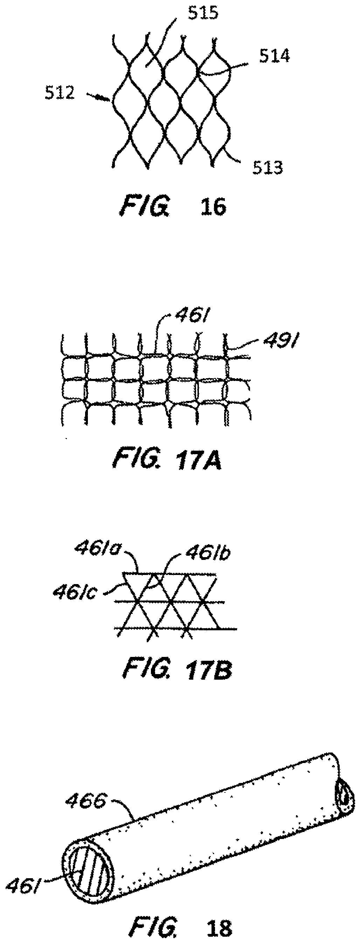

Intersecting filaments (FIG. 14C) may be used to form diamond shaped filter cells (FIG. 14A), as well as rectangular shaped filter cells 419 (FIGs. 14B). Multiple strand patterns may also be used such as the three strand 461a, 461b and 461c array illustrated in FIG. 17B. Intersecting filaments may also be knotted, tied or otherwise joined 468 (FIGs. 15A and 15E). Intersecting filaments may form the same or different filter cell shapes such as, for example, an elongated oval in FIG. 15C, one or more joined diamonds as in FIG. 15B and an array of joined polygons as in FIG. 15D. In one embodiment, a filter cell is defined by at least three intersecting filaments 461. The filter element 461 may be formed from any of a wide variety of acceptable materials that are biocompatible and will filter debris. For example, filaments, lines and strands described herein may be in the form of a multifilament suture, a monofilament suture a ribbon, a polymer strand, a metallic strand or a composite strand. Additionally, filaments, lines and strands described herein may be formed from expanded polytetrafluoroethylene (ePTFE), polytetrafluoroethylene (PTFE), Poly(ethylene terephthalate) (PET), Polyvinylidene fluoride (PVDF), tetrafluoroethylene-co-hexafluoropropylene (FEP), or poly(fluoroalkoxy) (PFA), other suitable medical grade polymers, other biocompatible polymers and the like.

The joined polygons may have any of the shapes illustrated in FIGs. 20A-20F. It is to be appreciated that filter cells may have any, one or more, or hybrid combinations of shapes such as, for example, circular (FIG. 20A), polygonal (FIG. 20B), oval (FIG. 20C), triangular (FIG. 20D), trapezoidal or truncated conical (FIG. 20E).

In addition, the material capture structure may have filter cells formed by extruding a material into a material capture structure. FIG. 16 illustrates an exemplary filtering structure 512 where a material is extruded into strands 513 that are joined 514 and spaced apart for form one of more filter cells 515. In one embodiment, the strands are extruded from Polypropylene material, forming diamond shaped filter cells approximately 4 mm in height and 3 mm in width.

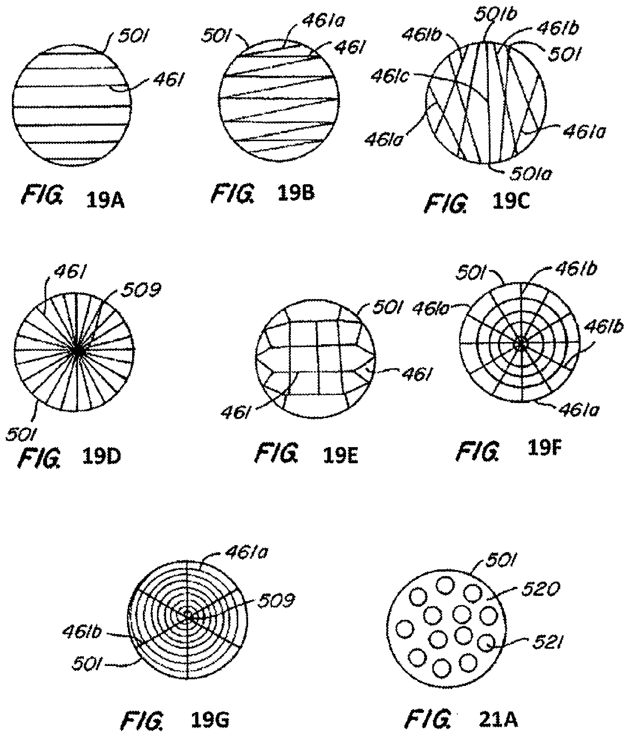

FIGs. 19A-23B illustrate several different filtering structure configurations. For simplicity of illustration, the filtering material is shown attached to a circular frame 501. It is to be appreciated that the circular frame 501 represents any of the various open loop, rounded frame or other support frames described herein. FIG. 19B adds an additional transverse filaments 461*a* at an angle to the filaments 461. FIG. 19C illustrates a plurality of filaments 461*a* extending up from the frame bottom 501 a about a central filament 461*c* and a plurality of filaments 461*b* extending down from the frame top 501*b* about a central filament 461c. In this illustrative embodiment, the filaments 461*a*,*b* are arranged symmetrically about the central filament 461*c*. Other non-symmetrical configurations are possible. More than one central filament 461*c* may be used to form a variety of different size and shaped polygonal filter cells (e.g., FIG. 19E).

Filaments may also be arranged using a variety of radial patterns. For example, multiple filaments 461 may from a common point 509 out the edge of frame 501. In some embodiments, the common point is central to the frame 501 (FIG. 19D) and in other embodiments the common point 509 is in a different, non-central location. The sectors formed by the multiple filaments (FIG. 19D) may be further divided into multiple filter cell segments by winding a filament 461*a* about and across segment filaments 461*b*. In contrast to a single filament spirally out from the point 509 as in FIG. 19G, the segmented filter cells in FIG. 19F are formed by attaching single filament 461*a* to the segment filaments 461*b*.

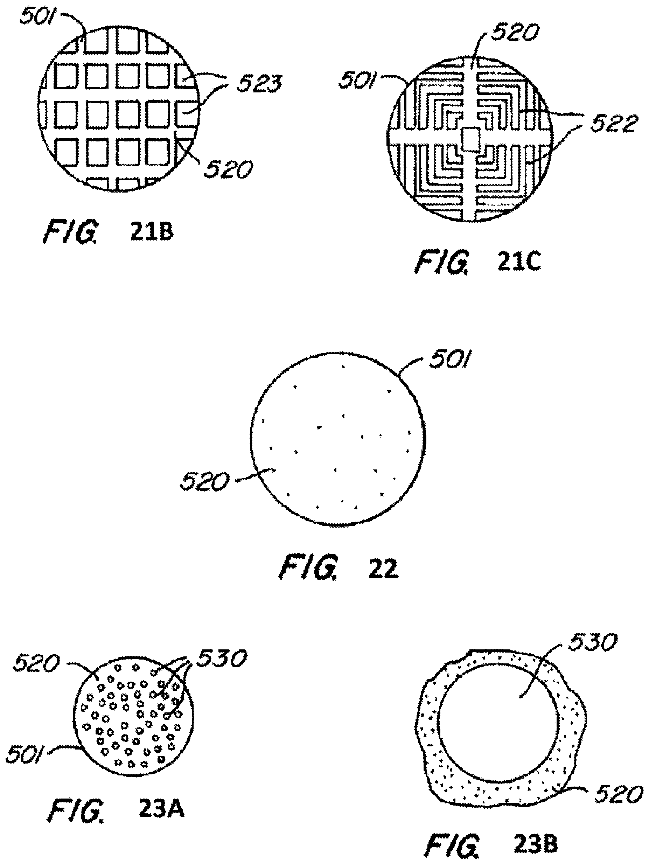

FIGs. 21A-C and FIG. 22 illustrate the use of a sheet of material 520 to form a filter structure. The material 520 may have any of a variety of shapes formed in it using any suitable process such as punching, piercing, laser cutting and the like. FIG. 21A illustrates a circular pattern 521 formed in material 520. FIG. 21B illustrates a rectangular pattern 523 formed in material 520. FIG. 21C illustrates a complex pattern 522 cut into material 522. It is to be appreciated that the material 520 may also be placed in the frame 501 without any pattern (FIG. 22). The illustrative embodiment of FIG. 22 may be useful for occluding the flow within a lumen. Suitable materials 520 for an occlusion application include for example, wool, silk polymer sheets, other material suited to prevent blood flow in a lumen when extended across a lumen and the like. Additionally, the filter material 520 may be a porous material having pores 530 (FIG. 23A). The material 520 may be selected based on the average size of individual pores 530 (FIG. 23B) depending upon the procedure or use of the filter device. For example, the material 520 may be any of the porous materials using in existing distal protection and embolic protection devices. In general, a wide variety of pore 530 sizes are available and may range from 0.254 mm to 7.62 mm (0.011" to 0.3"). Other pore sizes are also available depending upon the material 520 selected.

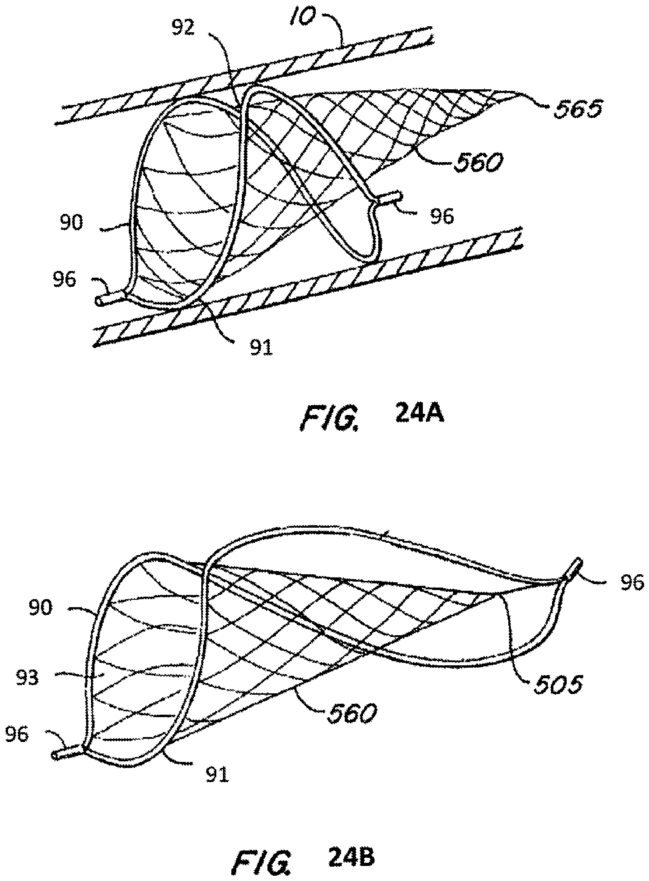

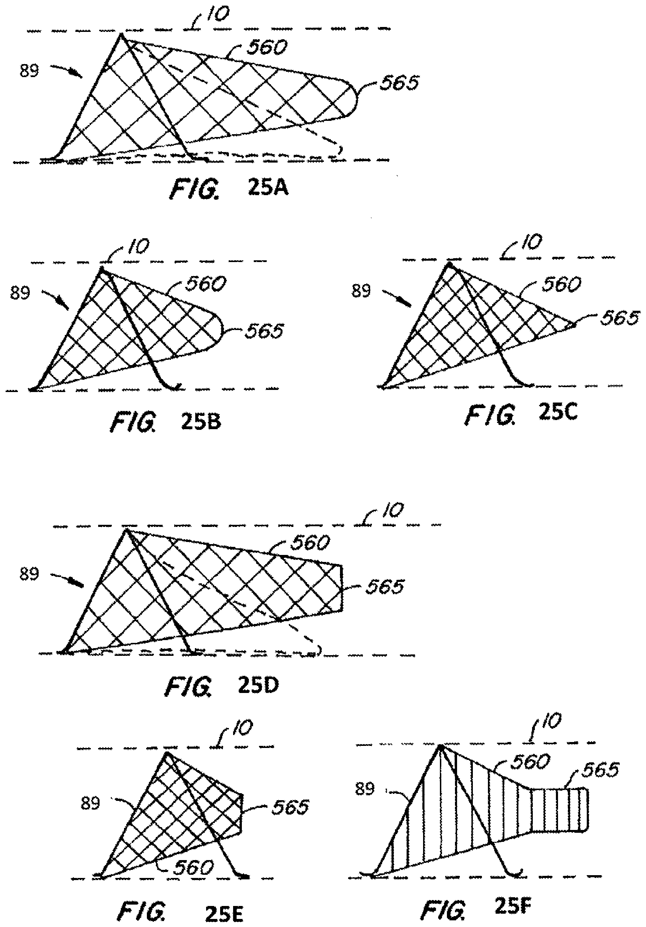

FIGs. 14-25F illustrate the use of nets or other web structures within the filtering device. The various net structure embodiments described herein are used as material capture structures within filter device embodiments of the present invention. Each of these alternative is illustrated in a support structure similar to that of device 89 in FIG. 1I and elsewhere; however, any of the alternatives can also be used with all of the embodiments of filter designs disclosed herein. When deployed within the lumen 10, the material capture structure 560 has a defined shape such as a cone with a discrete apex 565 (FIG. 24A). In this embodiment, the net structure is long enough to contact the sidewall of the lumen 10 when deployed in the lumen 10. Alternatively, the apex 565 may be attached to the end 96 to keep the net 560 in the lumen flow path and out of contact with the lumen sidewall (FIG. 24B). The net 565 may also have a rounded apex 565 (FIG. 25A) or a truncated cone (flat bottom) (FIG. 25D). Alternatively, the net 560 may a discrete apex 565 so short that it will not contact the lumen sidewall when deployed (FIG. 25B). The short net may also have a rounded apex 565 (FIG. 25B), a flat apex (FIG. 25E) or a sharp apex (FIG. 25C). In addition, the net 560 may have a compound apex 565 (FIG. 25F).

## Claims

1. An endoluminal filter (200), comprising:
a support frame (202) with a first wall portion forming a first loop (204) and a second wall portion forming a second loop (206), the first wall portion and second wall portion configured such that the first wall portion does not crossover the second wall portion;
a material capture structure (212,216) extending between the first wall portion of the first loop; and **characterized in that** the support frame comprises a microtruss structure (318a,318b) having a metallic lattice.

2. The endoluminal filter according to claim 1, wherein the support frame (202) comprises a drug embedded in interstitial spaces of the microtruss structure (318a,318b).

3. The endoluminal filter according to claim 1, further comprising a retrieval feature (214).

4. The endoluminal filter according to claim 1, wherein at least one tissue anchor (120) is formed on a surface of the first wall portion or second wall portion.

5. The endoluminal filter according to claim 1, wherein the support frame is laser machined from a tubular structure.

6. The endoluminal filter according to claim 1, wherein the support frame is made using a 3D printer.

7. The endoluminal filter according to claim 1, wherein the first wall portion and the second wall portion include one or more openings configured to engage with the material capture structure (212,216).

8. The endoluminal filter according to claim 1, wherein the first wall portion and/or the second wall portion includes a sinusoidal shape.

9. The endoluminal filter according to claim 8, wherein the material capture structure (212,216) extends between the first wall portion having the sinusoidal shape.

10. The endoluminal filter according to claim 1, further comprising a rigid connection member (122,226) between the support frame and an opposing side of the support frame.

11. The endoluminal filter according to claim 10, wherein the rigid connection member (122) joins the support frame and opposing side of the support frame at a position where the distance between the support frame and opposing side of the support frame is at a minimum.

12. The endoluminal filter according to claim 10, further comprising a retrieval member projecting from the rigid connection member.

13. The endoluminal filter according to claim 1, further comprising an elongate third portion between the first loop and second loop.

14. The endoluminal filter according to claim 1, the support frame further comprising a third wall portion forming a third loop (209).

15. The endoluminal filter according to any of claim 1, wherein the endoluminal filter is made out of a single material.

## Patentansprüche

1. Endoluminales Filter (200), umfassend:
einen Tragrahmen (202) mit einem ersten Wandabschnitt, der eine erste Schleife (204) bildet, und einem zweiten Wandabschnitt, der eine zweite Schleife (206) bildet, wobei der erste Wandabschnitt und ein zweiter Wandabschnitt so konfiguriert sind, dass der erste Wandabschnitt den zweiten Wandabschnitt nicht überkreuzt;
eine Materialeinfangstruktur (212, 216), die sich zwischen dem ersten Wandabschnitt der ersten Schleife erstreckt; und **dadurch gekennzeichnet ist, dass** der Tragrahmen eine Mikrostruktur (318a, 318b) umfasst, die ein Metallgitter aufweist.

2. Endoluminales Filter nach Anspruch 1, wobei der Tragrahmen (202) ein Arzneimittel umfasst, das in Zwischenräume der Mikrotrussstruktur (318a, 318b) eingebettet ist.

3. Endoluminales Filter nach Anspruch 1, weiter ein Entnahmemerkmal (214) umfassend.

4. Endoluminales Filter nach Anspruch 1, wobei mindestens ein Gewebeanker (120) auf einer Oberfläche des ersten Wandabschnitts oder zweiten Wandabschnitts ausgebildet ist.

5. Endoluminales Filter nach Anspruch 1, wobei der Tragrahmen aus einer rohrförmigen Struktur laserbearbeitet ist.

6. Endoluminales Filter nach Anspruch 1, wobei der Tragrahmen unter Verwendung eines 3D-Druckers hergestellt ist.

7. Endoluminales Filter nach Anspruch 1, wobei der erste Wandabschnitt und der zweite Wandabschnitt eine oder mehrere Öffnungen beinhalten, die zum Eingriff mit der Materialaufnahmestruktur (212, 216) konfiguriert sind.

8. Endoluminales Filter nach Anspruch 1, wobei der erste Wandabschnitt und/oder der zweite Wandabschnitt eine Sinusform beinhalten.

9. Endoluminales Filter nach Anspruch 8, wobei sich die Materialaufnahmestruktur (212, 216) zwischen dem ersten Wandabschnitt, der die Sinusform aufweist, erstreckt.

10. Endoluminales Filter nach Anspruch 1, weiter ein starres Verbindungselement (122, 226) zwischen dem Tragrahmen und einer gegenüberliegenden Seite des Tragrahmens umfassend.

11. Endoluminales Filter nach Anspruch 10, wobei das starre Verbindungselement (122) mit dem Tragrahmen und der gegenüberliegenden Seite des Tragrahmens an einer Position verbunden ist, an der der Abstand zwischen dem Tragrahmen und der gegenüberliegenden Seite des Tragrahmens minimal ist.

12. Endoluminales Filter nach Anspruch 10, weiter ein Entnahmeelement umfassend, das von dem starren Verbindungselement hinausragt.

13. Endoluminales Filter nach Anspruch 1, weiter einen länglichen dritten Abschnitt zwischen der ersten Schleife und der zweiten Schleife umfassend.

14. Endoluminales Filter nach Anspruch 1, wobei der Tragrahmen weiter einen dritten Wandabschnitt umfasst, der eine dritte Schleife (209) bildet.

15. Endoluminales Filter nach einem der Ansprüche 1, wobei der endoluminale Filter aus einem einzigen Material hergestellt ist.

## Revendications

1. Filtre endoluminal (200), comprenant :
un cadre de support (202) avec une première portion paroi formant une première boucle (204) et une deuxième portion paroi formant une deuxième boucle (206), la première portion paroi et la deuxième portion paroi étant configurées de sorte que la première portion paroi ne croise pas la deuxième portion paroi ;
une structure de capture de matières (212, 216) s'étendant entre la première portion paroi de la première boucle ; et **caractérisé en ce que** le cadre de support comprend une structure en micro-treillis (318a, 318b) ayant un réseau métallique.

2. Filtre endoluminal selon la revendication 1, dans lequel le cadre de support (202) comprend un médicament incorporé dans des espaces interstitiels de la structure en micro-treillis (318a, 318b).

3. Filtre endoluminal selon la revendication 1, comprenant en outre un élément de récupération (214).

4. Filtre endoluminal selon la revendication 1, dans lequel au moins un ancrage tissulaire (120) est formé sur une surface de la première portion paroi ou de la deuxième portion paroi.

5. Filtre endoluminal selon la revendication 1, dans lequel le cadre de support est usiné au laser à partir d'une structure tubulaire.

6. Filtre endoluminal selon la revendication 1, dans lequel le cadre de support est fabriqué en utilisant une imprimante 3D.

7. Filtre endoluminal selon la revendication 1, dans lequel la première portion paroi et la deuxième portion paroi incluent une ou plusieurs ouvertures configurées pour venir en prise avec la structure de capture de matières (212, 216).

8. Filtre endoluminal selon la revendication 1, dans lequel la première portion paroi et/ou la deuxième portion paroi inclut une forme sinusoïdale.

9. Filtre endoluminal selon la revendication 8, dans lequel la structure de capture de matières (212, 216) s'étend entre la première portion paroi ayant la forme sinusoïdale.

10. Filtre endoluminal selon la revendication 1, comprenant en outre un organe de liaison rigide (122, 226) entre le cadre de support et un côté opposé du cadre de support.

11. Filtre endoluminal selon la revendication 10, dans lequel l'organe de liaison rigide (122) relie le cadre de support et un côté opposé du cadre de support au niveau d'une position où la distance entre le cadre de support et un côté opposé du cadre de support est minimale.

12. Filtre endoluminal selon la revendication 10, comprenant en outre un organe de récupération faisant saillie depuis l'organe de liaison rigide.

13. Filtre endoluminal selon la revendication 1, comprenant en outre une troisième portion allongée entre la première boucle et la deuxième boucle.

14. Filtre endoluminal selon la revendication 1, le cadre de support comprenant en outre une troisième portion paroi formant une troisième boucle (209).

15. Filtre endoluminal selon l'une quelconque de la revendication 1, dans lequel le filtre endoluminal est fabriqué à partir d'un matériau unique.
